# FASCICULE DE BREVET EUROPEEN

(11) **EP 2 344 870 B1**
(45) Date de publication et mention de la délivrance du brevet: **04.07.2012**
(21) Numéro de dépôt: 09783781.9
(22) Date de dépôt: 06.10.2009
(51) Int. Cl.: G01N 27/407

(54) **DISPOSITIF DE DÉTERMINATION DE LA CONCENTRATION EN MONOXYDE DE CARBONE ET PROCÉDÉ**
VORRICHTUNG ZUR BESTIMMUNG DER KOHLENMONOXIDKONZENTRATION UND DAMIT VERBUNDENES VERFAHREN
DEVICE FOR DETERMINING CARBON MONOXIDE CONCENTRATION AND RELATED METHOD

(30) Priorité: 10.10.2008 FR 0856878
(43) Date de publication de la demande: 20.07.2011
(73) Titulaire: Commissariat à l'Énergie Atomique et aux Énergies Alternatives, 75015 Paris (FR)
(72) Inventeur: LEMAIRE, Olivier, F-38490 Les Abrets (FR); FRANCO, Alejandro, F-38320 Eybens (FR); GUILLET, Nicolas, F-26300 Chatuzange Le Goubet (FR)
(74) Mandataire: Ilgart, Jean-Christophe
(86) Numéro de dépôt international: PCT/EP2009/062960
(87) Numéro de publication internationale: WO 2010/040739

(56) Documents cités:
- EP-A- 1 767 930
- US-B1- 6 488 836
- FARRELL ET AL: "Experimental and modelling studies of CO poisoning in PEM fuel cells" JOURNAL OF POWER SOURCES, ELSEVIER, AMSTERDAM, NL, vol. 171, no. 2, 8 septembre 2007 (2007-09-08), pages 282-293, XP022238025 ISSN: 0378-7753 cité dans la demande
- TURNER J E ET AL: "Oscillatory oxidation of co over Pd and Ir catalysts" SURFACE SCIENCE, NORTH-HOLLAND PUBLISHING CO, AMSTERDAM, NL, vol. 109, no. 3, 1 septembre 1981 (1981-09-01), pages 591-604, XP025750045 ISSN: 0039-6028 [extrait le 1981-09-01]

## Description

### DOMAINE TECHNIQUE

La présente invention se rapporte au domaine de la détermination de la concentration en monoxyde de carbone (CO) dans un gaz contenant de l'hydrogène, notamment lorsque ce gaz est destiné à alimenter la partie anodique des piles à combustible du type à membrane échangeuse de protons (*PEMFC*).

### ETAT DE LA TECHNIQUE ANTERIEURE

Une pile à combustible comporte généralement un empilement de cellules élémentaires, au sein desquelles a lieu une réaction électrochimique entre deux réactifs qui sont introduits de manière continue.

Le combustible, par exemple l'hydrogène, est amené au contact de l'anode alors que le comburant, généralement l'oxygène, est amené au contact de la cathode.

L'anode et la cathode sont séparées par un électrolyte, de type membrane échangeuse de protons.

Dans le cas d'une pile hydrogène / oxygène, l'anode est le lieu de l'oxydation de l'hydrogène, et à la cathode est réalisée la réduction de l'oxygène. On assiste alors à une réaction électrochimique qui génère de l'énergie électrique.

Un catalyseur, par exemple des grains de platine, est généralement présent à l'anode pour améliorer le rendement de la demi-réaction électrochimique.

Cependant, les sites catalytiques sont particulièrement sensibles au CO qui a tendance à venir s'y accumuler. Ainsi, il est connu qu'une concentration en CO de quelques ppm suffit à empoisonner les sites catalytiques.

Cet empoisonnement se traduit notamment par une augmentation sensible du potentiel anodique, qui induit une chute de la tension aux bornes de la pile. Les performances de la pile sont alors fortement diminuées.

Or, l'hydrogène utilisé est couramment obtenu par reformage d'un composé hydrocarboné (pétrole, gaz naturel, charbon, biocarburant...). Par cette méthode, on obtient un gaz de reformat riche en hydrogène, mais contenant également une concentration en CO qui peut aller de quelques dizaines de ppm à quelques pourcents.

Aussi, il est important de pouvoir déterminer rapidement la concentration en CO présent dans un gaz destiné à alimenter l'anode d'une pile à combustible, à l'aide d'un dispositif présentant une grande simplicité de mise en ouvre.

Le brevet US6488836 décrit un dispositif électrochimique comprenant une électrode de détection séparée d'une contre-électrode par une membrane électrolytique solide, chaque électrode étant connectée à une unité de commande.

Lorsque l'électrode de détection est en contact avec du gaz contenant de l'hydrogène et du CO, une source de tension applique une différence de potentiel entre les deux électrodes de manière à provoquer l'oxydation de l'hydrogène au niveau de l'électrode de détection, le transfert des protons au travers de l'électrolyte et la réduction des protons au niveau de la contre-électrode.

La source de tension fait également varier la différence de potentiel par une fonction créneau. Plus précisément, le potentiel appliqué à l'électrode de détection alterne entre un potentiel faible autorisant l'adsorption du CO par l'électrode de détection et un potentiel élevé entraînant l'oxydation du CO.

L'unité de commande mesure, via un ampèremètre, la chute de courant induite par la variation de tension appliquée, calcule le taux de diminution du courant correspondant, puis détermine la concentration en CO à l'aide d'une courbe de calibration indiquant la concentration en CO en fonction du taux de diminution.

Ce dispositif présente cependant l'inconvénient d'avoir à déterminer au préalable un certain nombre de paramètres destinés à commander de manière contrôlée les phases d'adsorption et de désorption du CO par l'électrode de détection, à l'aide de moyens électriques. Il est en effet nécessaire de définir la fréquence et les valeurs limites minimale et maximale de la variation de tension appliquée.

Par ailleurs, ces paramètres peuvent dépendre des conditions opératoires, des caractéristiques physiques de l'électrode de détection, de la surface de la zone active, ainsi que de la concentration en CO dans le gaz. Le fonctionnement du dispositif n'est alors pas optimisé.

### EXPOSÉ DE L'INVENTION

Le but de la présente invention est de proposer un dispositif de détermination de la concentration en CO présent dans un gaz contenant de l'hydrogène, présentant une structure et un fonctionnement simplifiés.

Pour ce faire, l'invention a pour objet un dispositif de détermination de la concentration en CO présent dans un gaz contenant de l'hydrogène, comprenant une électrode de détection destinée à être en contact avec ledit gaz, et une contre-électrode, chacune étant en contact avec un électrolyte.

Selon l'invention, le dispositif comprend :
- une source de courant pour délivrer un courant d'intensité déterminée entre l'électrode de détection et la contre-électrode de manière à générer, à l'électrode de détection, un potentiel électrique oscillant entre deux valeurs limites du fait de l'adsorption et de la désorption du CO au niveau de ladite électrode de détection,
- des moyens de mesure dudit potentiel, et
- une unité de calcul pour déterminer la concentration en CO, connectée à ladite source de courant et auxdits moyens de mesure dudit potentiel, comprenant des moyens de calcul pour calculer un paramètre caractéristique des oscillations dudit potentiel, et des moyens de détermination pour déterminer la concentration en CO à partir dudit paramètre caractéristique calculé et de l'intensité du courant appliqué.

Le dispositif selon l'invention présente ainsi une structure et un fonctionnement simplifiés.

En effet, l'application d'un courant électrique entre les électrodes (mode galvanostatique) par le dispositif selon l'invention fait apparaître des oscillations spontanées du potentiel électrique de l'électrode de détection correspondant à une succession de phases d'adsorption et de désorption de CO au niveau de l'électrode de détection. Ces oscillations sont alors caractérisées par un paramètre pertinent qui permet d'obtenir, en fonction de l'intensité du courant électrique, la concentration en CO présent dans ledit gaz.

Il n'est donc pas nécessaire d'avoir des moyens électriques destinés à commander de manière contrôlée les phases d'adsorption et de désorption du CO par l'électrode de détection. Il n'est pas non plus nécessaire de déterminer au préalable des paramètres électriques pour commander de manière contrôlée ces phases, comme dans le dispositif selon l'art antérieur.

Ces oscillations permettent également de régénérer l'électrode de détection sans intervention particulière de l'utilisateur.

Le paramètre caractéristique est avantageusement la pente à mi-hauteur calculée lorsque ledit potentiel augmente du potentiel inférieur au potentiel supérieur.

Lesdits moyens de détermination comprennent avantageusement une carte mémoire contenant une table de données prédéterminées indiquant la concentration en CO en fonction dudit paramètre caractéristique et de la densité de courant.

Selon un mode de réalisation, lesdits moyens de mesure du potentiel comprennent un voltmètre pour mesurer la différence de potentiel entre l'électrode de détection et la contre-électrode.

Selon une variante, lesdits moyens de mesure du potentiel comprennent un voltmètre pour mesurer la différence de potentiel entre l'électrode de détection et une électrode de référence.

L'électrolyte peut comprendre une membrane solide présentant une conductivité protonique et/ou une solution électrolytique.

De préférence, ladite électrode de détection présente une surface en contact avec l'électrolyte sur laquelle est disposé un catalyseur électrochimique susceptible d'adsorber du CO.

L'invention porte également sur un procédé de détermination de la concentration en CO présent dans un gaz contenant de l'hydrogène.

Selon l'invention, le procédé comprend les étapes suivantes :
- mettre en contact ledit gaz avec une électrode de détection ;
- délivrer un courant électrique d'intensité déterminée entre ladite électrode de détection et une contre-électrode, lesdites électrodes étant en contact avec un électrolyte, de manière à générer, à l'électrode de détection, un potentiel électrique oscillant entre deux valeurs limites du fait de l'adsorption et de la désorption du CO au niveau de ladite électrode de détection ;
- mesurer ledit potentiel ;
- calculer un paramètre caractéristique des oscillations dudit potentiel ;
- déterminer la concentration en CO à partir dudit paramètre caractéristique calculé et de l'intensité du courant appliqué.

De préférence, le calcul du paramètre caractéristique consiste à calculer la pente à mi-hauteur lorsque ledit potentiel augmente du potentiel inférieur au potentiel supérieur.

La mesure dudit potentiel peut consister à mesurer la différence de potentiel entre ladite électrode de détection et ladite contre-électrode, ou à mesurer la différence de potentiel entre ladite électrode de détection et une électrode de référence.

Par ailleurs, l'étape de détermination de la concentration en CO peut consister à comparer les valeurs du paramètre caractéristique calculé et de l'intensité du courant appliqué à une table de données prédéterminées indiquant la concentration en CO en fonction du paramètre caractéristique et de l'intensité du courant.

D'autres avantages et caractéristiques de l'invention apparaîtront dans la description détaillée non limitative ci-dessous.

### BRÈVE DESCRIPTION DES DESSINS

On décrira à présent, à titre d'exemples non limitatifs, des modes de réalisation de l'invention, en se référant aux dessins annexés, parmi lesquels :
La figure 1 est une représentation schématique d'un dispositif de détermination de la concentration en CO selon un premier mode de réalisation de l'invention, comprenant deux électrodes séparées l'une de l'autre par un électrolyte membranaire solide ;
La figure 2A est une courbe d'oscillation du potentiel électrique de l'électrode de détection sur laquelle est indiquée la pente à mi-hauteur, alors que la figure 2B est une table de données de référence reliant la concentration en CO à la pente à mi-hauteur du potentiel de l'électrode de détection, pour plusieurs densités de courant ;
La figure 3 est une représentation schématique d'un dispositif de détermination de la concentration en CO selon un second mode de réalisation de l'invention, comprenant trois électrodes baignant dans une solution électrolytique ;
La figure 4 est une vue en coupe longitudinale d'un support d'électrode mis en oeuvre dans le second mode de réalisation représenté sur la figure 3.

### EXPOSÉ DÉTAILLÉ D'UN MODE DE RÉALISATION PREFERE

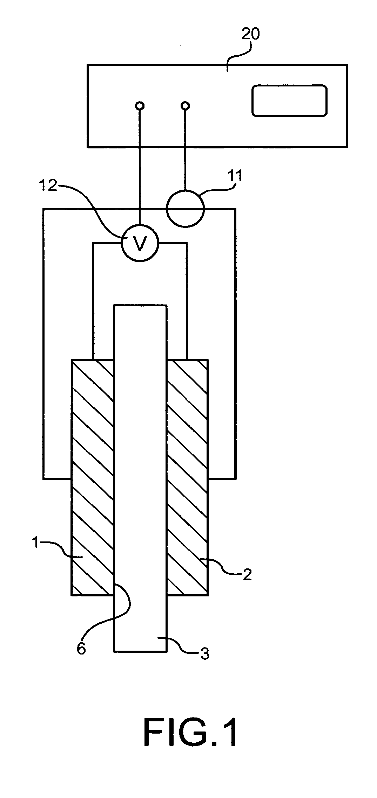

En référence à la figure 1, un dispositif de détermination de la concentration en CO selon un premier mode de réalisation de l'invention comprend une électrode de détection 1 séparée d'une contre-électrode 2 par un électrolyte solide 3 sous forme d'une membrane polymère échangeuse de protons. Chaque électrode 1, 2 comporte une surface en contact avec ladite membrane 3.

L'électrode de détection 1 peut être un milieu poreux électriquement conducteur, par exemple un milieu carboné. Elle comporte une surface sur laquelle est déposé un catalyseur électrochimique, par exemple du platine ou un alliage de platine, définissant ainsi une zone active 6. Ladite zone active 6 est en contact avec l'électrolyte 3. Les sites catalytiques de la zone active 6 sont susceptibles d'être empoisonnés par le CO.

Un gaz contenant de l'hydrogène et du CO, par exemple un gaz de reformage, est amené au contact de l'électrode de détection 1, par exemple par un conduit d'amenée non représenté. Il diffuse au travers du milieu poreux pour venir jusqu'à la zone active 6.

La contre-électrode 2 présente, de préférence, une structure identique à celle de l'électrode de détection 1.

La membrane 3 peut être une membrane polymère du type NAFION^{®} commercialisée par DuPont de Nemours.

Une source de courant 11 est connectée aux deux électrodes 1, 2 et fait circuler un courant d'intensité voulue entre celles-ci, comprise entre quelques milliampères par centimètre carré et quelques centaines de milliampères par centimètre carré.

Des moyens de mesure 12 du potentiel de l'électrode de détection sont prévus. Dans ce mode de réalisation, les moyens de mesure 12 du potentiel comprennent un voltmètre pour mesurer la différence de potentiel entre l'électrode de détection 1 et la contre-électrode 2.

Lorsque le gaz diffuse jusqu'à la zone active 6 de l'électrode de détection 1, et que la source de courant 11 délivre un courant électrique, une réaction d'oxydation de l'hydrogène est alors réalisée au niveau de l'électrode de détection 1. Les protons traversent la membrane 3 jusqu'à la contre-électrode 2 où ils sont réduits électrochimiquement pour former de l'hydrogène. Les électrons circulent à l'extérieur jusqu'à la contre-électrode 2 où ils se recombinent avec les protons.

Le potentiel anodique présente alors des oscillations spontanées entre une valeur basse et une valeur haute, comme le montre l'article de Farrell et al. intitulé « Experimental and modelling studies of CO poisoning in PEM fuel cells », J. Power Sources, 171 (2007), 282-293. Chaque période d'oscillation comprend donc une phase d'augmentation du potentiel de l'électrode de détection 1 du fait de l'adsorption du CO sur l'électrode, suivie d'une phase de diminution du potentiel du fait de la désorption du CO. Ainsi, l'électrode de détection 1 se régénère automatiquement, c'est-à-dire se 'désempoisonne' du CO sans qu'il soit nécessaire de commander électriquement chaque phase d'adsorption et de désorption.

La source de courant 11 et le voltmètre 12 sont connectés à une unité de calcul 20 conçue pour déterminer la concentration en CO présent dans ledit gaz.

Elle comprend des moyens de calcul pour calculer un paramètre caractéristique des oscillations du potentiel mesuré de l'électrode de détection 1, un microprocesseur, une mémoire morte et de préférence une mémoire vive, ou carte mémoire.

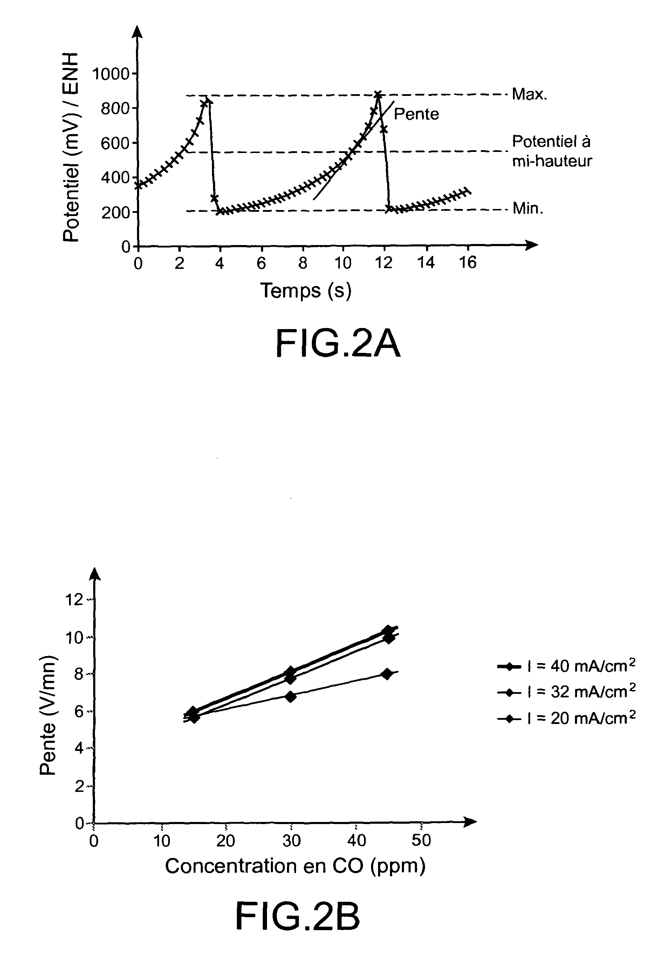

Le paramètre caractéristique est avantageusement la pente à mi-hauteur calculée dans la phase d'adsorption, c'est-à-dire lorsque le potentiel de l'électrode de détection 1 augmente d'une valeur limite basse à une valeur limite haute. La figure 2A est un exemple de courbe d'oscillation du potentiel électrique de l'électrode de détection 1 sur laquelle est indiquée la pente à mi-hauteur.

Bien entendu, la valeur retenue peut être la moyenne de la pente à mi-hauteur calculée sur plusieurs périodes d'oscillations, ou également la valeur convergée de la pente à mi-hauteur mesurée sur une pluralité de périodes.

Enfin, l'unité de calcul 20 est conçue pour déterminer la concentration en CO à partir du paramètre caractéristique calculé et de l'intensité du courant appliqué, à l'aide d'une table de données de référence préalablement déterminées, stockée dans la carte mémoire. La figure 2B est un exemple de table de données de référence reliant la concentration en CO à la pente à mi-hauteur du potentiel de l'électrode de détection 1, pour plusieurs densités de courant. De manière connue, la densité de courant correspond au rapport de l'intensité du courant appliqué sur la surface de la zone active 6 de l'électrode de détection 1.

Cette table de données de référence prédéterminées indique la concentration en CO en fonction du paramètre caractéristique et de l'intensité du courant. L'obtention de la table de données de référence est détaillée plus loin.

Le fonctionnement du dispositif selon le premier mode de réalisation de l'invention est le suivant.

On alimente l'électrode de détection 1 en gaz contenant de l'hydrogène et du CO.

L'hydrogène diffuse jusqu'à la zone active 6 de l'électrode de détection 1, tandis que le CO est adsorbé sur les sites catalytiques.

La source de courant 11 fait circuler un courant électrique entre l'électrode de détection 1 et la contre-électrode 2. La réaction d'oxydation de l'hydrogène a lieu au niveau de la zone active 6, et le potentiel de l'électrode de détection 1 présente des oscillations telles que décrites précédemment.

Le voltmètre 12 mesure la différence de potentiel entre l'électrode de détection 1 et la contre-électrode 2.

L'unité de calcul 20 enregistre la valeur de l'intensité du courant électrique appliqué, ainsi que l'évolution du potentiel de l'électrode de détection 1.

Les moyens de calcul calculent la pente à mi-hauteur des oscillations lors de la phase d'adsorption du potentiel électrique.

L'unité de calcul 20 détermine alors, par comparaison de la valeur de l'intensité du courant appliqué et celle de la pente à mi-hauteur à la table de données de référence, la concentration en CO présent dans le gaz.

Les données de référence peuvent être obtenues préalablement en utilisant un dispositif de détermination de la concentration en CO de préférence identique à celui de l'invention.

Un gaz, contenant de l'hydrogène et une concentration connue en CO, est amené au contact de l'électrode de détection 1.

Le dispositif mesure les oscillations du potentiel électrique de l'électrode de détection 1 et calcule la pente à mi-hauteur sur au moins une période d'oscillation, lors de la phase d'adsorption.

La valeur de la pente calculée et celle de l'intensité du courant appliqué, ainsi que la valeur de la concentration en CO correspondant, sont alors rentrées dans la table de données de référence.

L'opération est alors réitérée pour différentes intensités du courant électrique, ainsi que pour différentes concentrations en CO. En dehors des points de mesure, la concentration en CO peut être obtenue par extrapolation, par exemple linéaire.

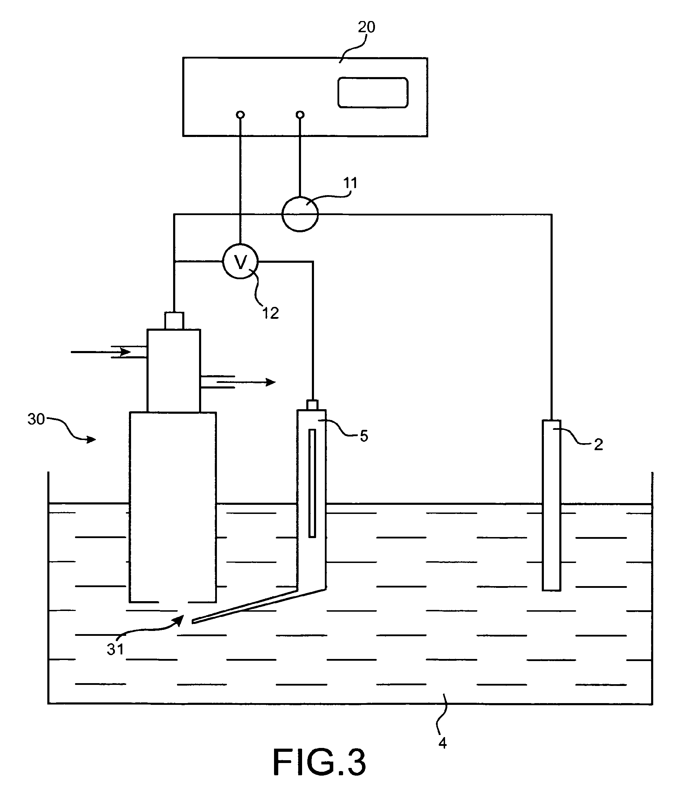

La figure 3 est une représentation schématique d'un dispositif de détermination de la concentration en CO selon un second mode de réalisation de l'invention, comprenant une électrode de détection 1, une contre-électrode 2 et une électrode de référence 5 en contact avec l'électrolyte ou avantageusement baignant dans une solution électrolytique.

Le dispositif selon ce mode de réalisation est sensiblement identique à celui du premier mode de réalisation, et en diffère par l'utilisation d'une électrode de référence qui permet d'obtenir des valeurs absolues de potentiel.

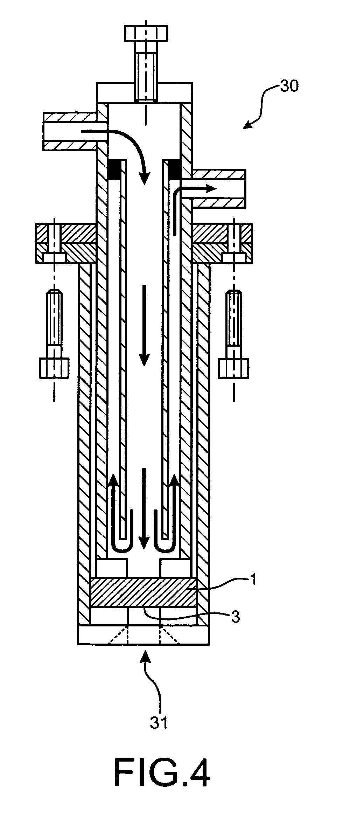

Dans ce mode de réalisation, l'électrode de détection 1 est, par exemple, disposée dans un support 30 d'électrode représenté sur la figure 4 et décrit en détail dans la demande de brevet FR2843635. Le support comporte une ouverture 31 en son extrémité inférieure, de manière à permettre la mise en contact de l'électrode de détection 1 avec la solution électrolytique 4.

L'électrode de détection 1 peut présenter une zone active de surface de l'ordre de 0,5 cm², chargé en catalyseur, par exemple 0,5 mg/cm² de platine.

Dans le but d'améliorer le fonctionnement du dispositif électrochimique, on met une membrane électrolytique solide, par exemple une membrane du type NAFION^{®}, en contact avec la zone active de l'électrode de détection sur une face, et avec le bain électrolytique en sa face opposée. Dans ce cas, il est avantageux que la membrane solide et le bain électrolytique 4 présentent une conductivité protonique sensiblement identique.

La contre-électrode 2 peut être un fil de platine baignant dans la solution.

L'électrode de référence 5 peut être une électrode à hydrogène munie d'un capillaire de Luggin dont l'extrémité est placée à proximité de l'électrode de détection 1, plus précisément à proximité de l'ouverture 31 du support 30, afin de minimiser l'influence de la chute ohmique. Un voltmètre 12 mesure la différence de potentiel entre l'électrode de détection 1 et l'électrode de référence 5. Dans le cas où le dispositif ne comprend pas d'électrode de référence, le voltmètre 12 mesure la différence de potentiel entre l'électrode de détection 1 et la contre-électrode 2.

L'électrolyte 4 peut être une solution d'acide sulfurique H₂SO₄, par exemple de 0,5 molaire.

Les électrodes 1, 2, 5 sont connectées à une unité de calcul 20 similaire à celle décrite précédemment. Le fonctionnement du dispositif selon ce second mode de réalisation de l'invention est sensiblement identique à celui du premier mode de réalisation.

L'utilisation du support 30 d'électrode de détection permet de contrôler de manière précise, simple et fiable les conditions opératoires, comme la température du gaz et son taux d'humidité. Il est également possible de changer facilement la surface de la zone active, et la nature du catalyseur, dans le but de déterminer les conditions optimales de fonctionnement du dispositif selon l'invention.

Par exemple, l'augmentation de la surface de la zone active permet d'augmenter la sensibilité du dispositif selon l'invention. De plus, l'utilisation d'un catalyseur plus tolérant au CO, comme par exemple l'alliage Pt-Ru, permet d'analyser des concentrations plus importantes en CO dans le gaz contenant de l'hydrogène.

Le dispositif selon l'invention peut être disposé en amont du compartiment anodique d'une pile à combustible, par exemple du type *PEMFC,* pour déterminer la concentration en CO présent dans le gaz alimentant ladite anode.

Cependant, l'utilisation dudit dispositif de détermination de la concentration en CO n'est pas limitée au domaine des piles à combustible, mais ledit dispositif peut être utilisé dans tout système de fabrication d'hydrogène ou de gaz à haute concentration d'hydrogène, particulièrement lorsque la pureté du gaz obtenu est un critère essentiel.

## Revendications

1. Dispositif de détermination de la concentration en CO présent dans un gaz contenant de l'hydrogène, comprenant une électrode de détection (1) destinée à être en contact avec ledit gaz, et une contre-électrode (2), chacune étant en contact avec un électrolyte (3 ; 4), caractérisé en qu'il comprend :
- une source de courant (11) pour délivrer un courant d'intensité déterminée entre l'électrode de détection (1) et la contre-électrode (2) de manière à générer, à l'électrode de détection (1), un potentiel électrique oscillant entre deux valeurs limites du fait de l'adsorption et de la désorption du CO au niveau de ladite électrode de détection (1),
- des moyens de mesure (12) dudit potentiel, et
- une unité de calcul (20) pour déterminer la concentration en CO, connectée à ladite source de courant (11) et auxdits moyens de mesure (12) dudit potentiel, comprenant des moyens de calcul pour calculer un paramètre caractéristique des oscillations dudit potentiel, et des moyens de détermination pour déterminer la concentration en CO à partir dudit paramètre caractéristique calculé et de l'intensité du courant appliqué.

2. Dispositif de détermination de concentration en CO selon la revendication 1, **caractérisé en ce que** le paramètre caractéristique est la pente à mi-hauteur calculée lorsque ledit potentiel augmente du potentiel inférieur au potentiel supérieur.

3. Dispositif de détermination de concentration en CO selon la revendication 1 ou 2, **caractérisé en ce que** lesdits moyens de détermination comprennent une carte mémoire contenant une table de données prédéterminées indiquant la concentration en CO en fonction dudit paramètre caractéristique et de la densité de courant.

4. Dispositif de détermination de concentration en CO selon l'une quelconque des revendications 1 à 3, **caractérisé en ce que** lesdits moyens de mesure (12) du potentiel comprennent un voltmètre pour mesurer la différence de potentiel entre l'électrode de détection (1) et la contre-électrode (2).

5. Dispositif de détermination de concentration en CO selon l'une quelconque des revendications 1 à 3, **caractérisé en ce que** lesdits moyens de mesure (12) du potentiel comprennent un voltmètre pour mesurer la différence de potentiel entre l'électrode de détection (1) et une électrode de référence (5).

6. Dispositif de détermination de concentration en CO selon l'une quelconque des revendications 1 à 5, **caractérisé en ce que** l'électrolyte (3 ; 4) comprend une membrane solide (3) présentant une conductivité protonique.

7. Dispositif de détermination de concentration en CO selon l'une quelconque des revendications 1 à 6, **caractérisé en ce que** l'électrolyte (3 ; 4) comprend une solution électrolytique (4).

8. Dispositif de détermination de concentration en CO selon l'une quelconque des revendications 1 à 7, **caractérisé en ce que** ladite électrode de détection (1) présente une surface (6) en contact avec l'électrolyte (3 ; 4) sur laquelle est disposé un catalyseur électrochimique susceptible d'adsorber du CO.

9. Procédé de détermination de la concentration en CO présent dans un gaz contenant de l'hydrogène, **caractérisé en ce qu'**il comprend les étapes suivantes :
- mettre en contact ledit gaz avec une électrode de détection (1) ;
- délivrer un courant électrique d'intensité déterminée entre ladite électrode de détection (1) et une contre-électrode (2), lesdites électrodes (1, 2) étant en contact avec un électrolyte (3 ; 4), de manière à générer, à l'électrode de détection (1), un potentiel électrique oscillant entre deux valeurs limites du fait de l'adsorption et de la désorption du CO au niveau de ladite électrode de détection (1) ;
- mesurer ledit potentiel ;
- calculer un paramètre caractéristique des oscillations dudit potentiel ;
- déterminer la concentration en CO à partir dudit paramètre caractéristique calculé et de l'intensité du courant appliqué.

10. Procédé selon la revendication 9, **caractérisé en ce que** le calcul du paramètre caractéristique consiste à calculer la pente à mi-hauteur lorsque ledit potentiel augmente du potentiel inférieur au potentiel supérieur.

11. Procédé selon la revendication 9 ou 10, **caractérisé en ce que** la mesure dudit potentiel revient à mesurer la différence de potentiel entre ladite électrode de détection (1) et ladite contre-électrode (2).

12. Procédé selon la revendication 9 ou 10, **caractérisé en ce que** la mesure dudit potentiel revient à mesurer la différence de potentiel entre ladite électrode de détection (1) et une électrode de référence (5).

13. Procédé selon l'une quelconque des revendications 9 à 12, **caractérisé en ce que** l'étape de détermination de la concentration en CO consiste à comparer les valeurs du paramètre caractéristique calculé et de l'intensité du courant appliqué à une table de données prédéterminées indiquant la concentration en CO en fonction du paramètre caractéristique et de l'intensité du courant.

## Claims

1. A device for determining the CO concentration in a gas containing hydrogen, comprising a detection electrode (1) intended to be in contact with said gas, and a counter electrode (2), each being in contact with an electrolyte (3; 4), **characterized in that** it comprises:
- a current source (11) to deliver a current with a predetermined intensity between the detection electrode (1) and the counter electrode (2) so as to generate, at the detection electrode (1), an electric potential fluctuating between two threshold values due to the adsorption and desorption of the CO at said detection electrode (1),
- means (12) for measuring said potential, and
- a calculating unit (20) to determine the CO concentration, connected to said current source (11) and to said means (12) for measuring the potential, comprising a calculating means for calculating a characteristic parameter of the fluctuations of said potential, and a determining means for determining the CO concentration from said calculated characteristic parameter and the intensity of the applied current.

2. The device for determining the CO concentration according to claim 1, **characterized in that** the characteristic parameter is the slope at mid-height calculated when said potential increases from the lower potential to the upper potential.

3. The device for determining the CO concentration according to claim 1 or 2, **characterized in that** said determining means comprises a memory board containing a predetermined data table indicating the CO concentration as a function of said characteristic parameter and the current density.

4. The device for determining the CO concentration according to any one of claims 1 to 3, **characterized in that** said means (12) for measuring the potential comprises a voltmeter for measuring the difference in potential between the detection electrode (1) and the counter electrode (2).

5. The device for determining the CO concentration according to any one of claims 1 to 3, **characterized in that** said means (12) for measuring the potential comprises a voltmeter for measuring the difference in potential between the detection electrode (1) and a reference electrode (5).

6. The device for determining the CO concentration according to any one of claims 1 to 5, **characterized in that** the electrolyte (3; 4) comprises a solid membrane (3) having a protonic conductivity.

7. The device for determining the CO concentration according to any one of claims 1 to 6, **characterized in that** the electrolyte (3; 4) comprises an electrolytic solution (4).

8. The device for determining the CO concentration according to any one of claims 1 to 7, **characterized in that** said detection electrode (1) has a surface (6) in contact with the electrolyte (3; 4) on which an electrochemical catalyst is positioned capable of adsorbing CO.

9. A method for determining the CO concentration present in a gas containing hydrogen, **characterized in that** it comprises the following steps:
- putting said gas in contact with a detection electrode (1);
- delivering an electric current with a predetermined intensity between said detection electrode (1) and a counter electrode (2), said electrodes (1, 2) being in contact with an electrolyte (3; 4), so as to generate, at the detection electrode (1), an electric potential fluctuating between two threshold values due to the adsorption and desorption of the CO at said detection electrode (1) ;
- measuring said potential;
- calculating a characteristic parameter of the fluctuations of said potential;
- determining the CO concentration from said calculated characteristic parameter and the intensity of the applied current.

10. The method according to claim 9, **characterized in that** the calculation of the characteristic parameter consists of calculating the slope at mid-height when said potential increases from the lower potential to the upper potential.

11. The method according to claim 9 or 10, **characterized in that** the measurement of said potential consists of measuring the difference in potential between said detection electrode (1) and said counter electrode (2).

12. The method according to claim 9 or 10, **characterized in that** the measurement of said potential consists of measuring the difference in potential between said detection electrode (1) and a reference electrode (5).

13. The method according to any one of claims 9 to 12, **characterized in that** the step for determining the CO concentration consists of comparing the values of the calculated characteristic parameter and the intensity of the current applied to a table of predetermined data indicating the CO concentration as a function of the characteristic parameter and the intensity of the current.

## Patentansprüche

1. Vorrichtung zur Bestimmung der Kohlenmonoxidkonzentration in einem Wasserstoff enthaltenden Gas, umfassend eine für den Kontakt mit dem genannten Gas bestimmte Detektionselektrode (1) und eine Gegenelektrode (2), wobei jede Kontakt hat mit einem Elektrolyten (3 ; 4),
**dadurch gekennzeichnet, dass** sie umfasst:
- eine Stromquelle (11) zur Lieferung eines Stroms von bestimmter Stärke zwischen der Detektionselektrode (1) und der Gegenelektrode (2), um so an der Detektionselektrode (1) ein elektrisches Potential zu erzeugen, das aufgrund der Adsorption und der Desorption des Kohlenmonoxids an der genannten Detektionselektrode (1) zwischen zwei Grenzwerten oszilliert,
- Messeinrichtungen (12) des genannten Potentials, und
- eine Recheneinheit (20) zur Bestimmung der CO-Konzentration, verbunden mit der genannten Stromquelle (11) und mit den genannten Messeinrichtungen (12) des genannten Potentials, Recheneinrichtungen zum Berechnen eines charakteristischen Parameters der Oszillationen des genannten Potentials, und Bestimmungseinrichtungen zum Bestimmen der CO-Konzentration aufgrund des genannten berechneten charakteristischen Parameters und der Stärke des angewendeten Stroms.

2. Vorrichtung zur Bestimmung der Kohlenmonoxidkonzentration nach Anspruch 1, **dadurch gekennzeichnet, dass** der charakteristische Parameter die Steilheit auf halber Höhe ist, berechnet wenn das genannte Potential vom unteren Potential zum oberen Potential ansteigt.

3. Vorrichtung zur Bestimmung der Kohlenmonoxidkonzentration nach Anspruch 1 oder 2, **dadurch gekennzeichnet, dass** die genannten Bestimmungseinrichtungen eine Speicherkarte umfassen, die eine Tabelle mit vorherbestimmten Daten enthält, welche die CO-Konzentration in Abhängigkeit von dem genannten berechneten charakteristischen Parameter und von der Stromstärke angibt.

4. Vorrichtung zur Bestimmung der Kohlenmonoxidkonzentration nach einem der Ansprüche 1 bis 3, **dadurch gekennzeichnet, dass** die genannten Messeinrichtungen (12) des Potentials ein Voltmeter zum Messen der Potentialdifferenz zwischen der Detektionselektrode (1) und der Gegenelektrode (2) umfasst.

5. Vorrichtung zur Bestimmung der Kohlenmonoxidkonzentration nach einem der Ansprüche 1 bis 3, **dadurch gekennzeichnet, dass** die genannten Messeinrichtungen (12) des Potentials ein Voltmeter zum Messen der Potentialdifferenz zwischen der Detektionselektrode (1) und einer Referenzelektrode (5) umfasst.

6. Vorrichtung zur Bestimmung der Kohlenmonoxidkonzentration nach einem der Ansprüche 1 bis 5, **dadurch gekennzeichnet, dass** der Elektrolyt (3 ; 4) eine feste Membran (3) mit einer Protonenleitfähigkeit umfasst.

7. Vorrichtung zur Bestimmung der Kohlenmonoxidkonzentration nach einem der Ansprüche 1 bis 6, **dadurch gekennzeichnet, dass** der Elektrolyt (3 ; 4) eine elektrolytische Lösung (4) umfasst.

8. Vorrichtung zur Bestimmung der Kohlenmonoxidkonzentration nach einem der Ansprüche 1 bis 7, **dadurch gekennzeichnet, dass** die genannte Detektionselektrode (1) eine Fläche (6) mit Kontakt zum Elektrolyten (3 ; 4) umfasst, auf der ein elektrochemischer Katalysator abgeschieden ist, der CO adsorbieren kann.

9. Verfahren zur Bestimmung der Kohlenmonoxidkonzentration in einem Wasserstoff enthaltenden Gas, **dadurch gekennzeichnet, dass** es die folgenden Schritte umfasst:
- Herstellen eines Kontakts zwischen dem genannten Gas und einer Detektionselektrode (1);
- Liefern eines elektrischen Stroms von bestimmter Stärke zwischen der Detektionselektrode (1) und der Gegenelektrode (2), wobei die genannten Elektroden (1, 2) Kontakt haben mit einem Elektrolyten (3 ; 4), um so an der Detektionselektrode (1) ein elektrisches Potential zu erzeugen, das aufgrund der Adsorption und der Desorption des Kohlenmonoxids an der genannten Detektionselektrode (1) zwischen zwei Grenzwerten oszilliert,
- Messen des genannten Potentials,
- Berechnen eines charakteristischen Parameters der Oszillationen des genannten Potentials,
- Bestimmen der CO-Konzentration aufgrund des genannten berechneten charakteristischen Parameters und der Stärke des angewendeten Stroms.

10. Verfahren nach Anspruch 9, **dadurch gekennzeichnet, dass** die Berechnung des charakteristischen Parameters darin besteht, die Neigung auf halber Höhe zu berechnen, wenn das genannte Potential vom unteren Potential zum oberen Potential ansteigt.

11. Verfahren nach Anspruch 9 oder 10, **dadurch gekennzeichnet, dass** das Messen des genannten Potentials darin besteht, die Potentialdifferenz zwischen der Detektionselektrode (1) und der Gegenelektrode (2) zu messen.

12. Verfahren nach Anspruch 9 oder 10, **dadurch gekennzeichnet, dass** das Messen des genannten Potentials darin besteht, die Potentialdifferenz zwischen der Detektionselektrode (1) und einer Referenzelektrode (5) zu messen.

13. Verfahren nach einem der Ansprüche 9 bis 12, **dadurch gekennzeichnet, dass** der Schritt zur Bestimmung der CO-Konzentration darin besteht, die Werte des berechneten Parameters und der angewendeten Stromstärke mit einer Tabelle mit vorherbestimmten Daten zu vergleichen, welche die CO-Konzentration in Abhängigkeit von dem genannten berechneten charakteristischen Parameter und von der Stromstärke angibt.
